# EUROPEAN PATENT APPLICATION

(11) **EP 3 293 534 A1**
(43) Date of publication of application: **14.03.2018**
(21) Application number: 17190444.4
(22) Date of filing: 11.09.2017
(51) Int. Cl.: G01R 33/48, G01R 33/422, G01R 33/30, G01T 1/16, A61B 6/00, A61B 6/03, A61B 5/00

(54) **COMBININING MAGNETIC RESONANCE IMAGING (MRI) AND POSITRON-EMISSION TOMOGRAPHY (PET)**

(30) Priority: 11.09.2016 US 201662393059 P
(71) Applicant: Aspect Imaging Ltd., 6085001 Shoham (IL)
(72) Inventor: DEZORAYEV, Aviad, 6085001 Shoham (IL); STERN, Yaki, 6085001 Shoham (IL); INBAR, Zahi, 6085001 Shoham (IL); REUVENI, Tobi, 6085001 Shoham (IL); BILU, Shaul, 6085001 Shoham (IL); NAVE, Ron, 6085001 Shoham (IL); BASH, Oren, 6085001 Shoham (IL)
(74) Representative: Pearl Cohen Zedek Latzer Baratz UK LLP

(57) **Abstract**

Systems and methods for simultaneous acquisition of MRI data and PET data of a subject are disclosed. The system can include a RF coil surrounding the subject and a PET detectors array surrounding the RF coil to generate the MRI data and the PET data of the subject, respectively. The system can include a RF shield positioned between the RF coil and the PET detectors array to prevent an interference between the RF coil and the PET detectors array to thereby allow simultaneous acquisition of the MRI data and the PET data without any one of the RF coil, the PET detectors array or the subject being moved during the imaging. The system can include an analysis unit to generate, based on the simultaneously acquired MRI data and PET data, combined PET-MRI images of the subject that include a spatially and temporally registered structural, functional and/or molecular data acquired under identical physiological conditions.

## Description

### CROSS REFERENCE TO RELATED APPLICATIONS

This application claims the benefit of and priority to U.S. Provisional Application No. 62/393,059 filed on September 11, 2016, which is incorporated herein by reference in its entirety.

### FIELD OF THE INVENTION

The present invention relates to the field of combined Positron Emission Tomography (PET) and Magnetic Resonance Imaging (MRI) systems.

### BACKGROUND OF THE INVENTION

Current combined Positron Emission Tomography (PET) and Magnetic Resonance Imaging (MRI) systems typically require moving at least one of MRI device's RF coil, PET detectors and/or a subject during an imaging procedure. For example, some systems house both a PET imaging device and a MRI imaging device. These systems can require that a subject be positioned relative to the PET detectors for a PET image to be taken. Subsequently, the same subject can be moved to be positioned within an MRI imaging device for an MRI image to be taken (or vice versa, the MRI image can be taken first then the PET). In these systems, fusing the PET image and the MRI image can result in erroneous results due to, for example, the subject's position during PET imaging being different than the subject's position during MRI imaging.

Another difficulty with current systems is that a state of the subject may be different during the PET image and the MRI image due to, for example, a delay between taking the PET image and the MRI image. The delay between taking the two images can also cause erroneous results. Therefore, it can be desirable to provide simultaneous PET and MRI imaging of the subject.

### SUMMARY OF THE INVENTION

One aspect of the present invention provides a system for combined magnetic resonance imaging (MRI) and Positron-Emission Tomography (PET). The system can include: at least one magnet to generate a magnetic field within a predetermined measurement volume; a radiofrequency (RF) coil having a RF coil bore that at least partly overlaps the predetermined measurement volume and having a diameter to accommodate at least a portion of a subject, the RF coil to generate an electromagnetic field within the RF coil bore to excite nuclei within the at least portion of the subject and to receive signals emitted from the at least portion of the subject when the electromagnetic field is removed and the excited nuclei relax; a PET detectors array surrounding the RF coil and to detect gamma rays emitted by positron-emitting (PE) radionuclides within the subject; and a RF shield surrounding the RF coil and positioned between the RF coil and the PET detectors array.

In some embodiments, the system further includes an analysis unit in communication with at least one of the RF coil and the PET detectors array. The analysis unit can: generate, based on MRI data acquired by the RF coil, at least one MRI image of the at least portion of the subject; generate, based on PET data acquired by the PET detectors array, at least one PET image of the at least portion of the subject; and combine at least one of the MRI images with at least one of the PET images to thereby generate at least one combined PET-MRI image.

In some embodiments, the MRI device includes at least one of permanent magnets, superconductive magnets or a combination thereof to generate a magnetic field within the predetermined measurement volume.

In some embodiments, the RF coil and the detector array are positioned such that the subject, RF coil and the detector array are stationary during operation.

In some embodiments, the RF shield comprises at least one of a cylinder or a mesh made from a conductive material.

In some embodiments, the RF shield to form a Faraday cage to thereby provide RF shielding of the system.

In some embodiments, the RF coil and the PET detectors array are positioned concentrically or at a predetermined radial distance with respect to each other.

In some embodiments, the at least one MRI image and the at least one PET image includes at least one of a set of temporary sequential images, a set of spatial images or any combination thereof.

In some embodiments, the system is configured to perform the combining by at least one of registering, superimposing, fusing or any combination thereof of at least one of the MRI images and at least one of the PET images.

Another aspect of the present invention provides a method of generating at least one combined magnetic resonance imaging (MRI) image and Positron-Emission Tomography (PET) image. The method can include: preventing a radiofrequency (RF) radiation generated by a RF coil from interfering with operation of a PET detectors array and a RF radiation generated by the PET detectors array from interfering with operation the RF coil; generating, based on MRI data acquired by the RF coil, at least one MRI image of at least a portion of a subject; generating, based on PET data acquired by the PET detectors array, at least one PET image of the at least portion of the subject; and combining at least one of the MRI images with at least one of the PET images to thereby generate at least one combined PET-MRI image.

In some embodiments, the method further includes operating the RF coil and the detector array simultaneously or separately without any one of the RF coil, the PET detectors array or the subject being moved during an imaging procedure.

In some embodiments, the combining includes at least one of registering, superimposing, fusing or any combination thereof of at least one of the MRI images and at least one of the PET images.

Another aspect of the present invention provides a device for combined magnetic resonance imaging (MRI) and Positron-Emission Tomography (PET), which is operative in a MRI device. The device can include: a radiofrequency (RF) coil having a RF coil bore that at least partly overlaps a predetermined measurement volume of the MRI device and having a diameter to accommodate at least a portion of a subject, the RF coil to generate an electromagnetic field within the RF coil bore to excite nuclei within the at least portion of the subject and to receive signals emitted from the at least portion of the subject when the electromagnetic field is removed and the excited nuclei relax; a PET detectors array surrounding the RF coil and to detect gamma rays emitted by positron-emitting (PE) radionuclides within the subject; and a RF shield surrounding the RF coil and positioned between the RF coil and the PET detectors array.

In some embodiments, the device includes an analysis unit in communication with at least one of the RF coil, the PET detectors array and the MRI device. The analysis unit can: generate, based on MRI data acquired by the RF coil, at least one MRI image of the at least portion of the subject; generate, based on PET data acquired by the PET detectors array, at least one PET image of the at least portion of the subject; and combine at least one of the MRI images with at least one of the PET images to thereby generate at least one combined PET-MRI image.

In some embodiments, the MRI device includes at least one of permanent magnets, superconductive magnets or a combination thereof to generate a magnetic field within the predetermined measurement volume of the MRI device.

In some embodiments, the RF coil and the detector array are positioned such that the subject, RF coil and the detector array are stationary during operation.

In some embodiments, the RF shield includes at least one of a cylinder or a mesh made from a conductive material.

In some embodiments, the RF shield to form a Faraday cage to thereby provide RF shielding of the system.

In some embodiments, each of the at least one MRI image and the at least one PET image comprises at least one of a set of temporary sequential images, a set of spatial images or any combination thereof.

In some embodiments, the device is configured to perform the combining by at least one of registering, superimposing, fusing or any combination thereof of at least one of the MRI images and at least one of the PET images.

These, additional, and/or other aspects and/or advantages of the present invention are set forth in the detailed description which follows; possibly inferable from the detailed description; and/or learnable by practice of the present invention.

### BRIEF DESCRIPTION OF THE DRAWINGS

Non-limiting examples of embodiments of the disclosure are described below with reference to figures attached hereto that are listed following this paragraph. Dimensions of features shown in the figures are chosen for convenience and clarity of presentation and are not necessarily shown to scale.

The subject matter regarded as the invention is particularly pointed out and distinctly claimed in the concluding portion of the specification. The invention, however, both as to organization and method of operation, together with objects, features and advantages thereof, can be understood by reference to the following detailed description when read with the accompanied drawings. Embodiments of the invention are illustrated by way of example and not limitation in the figures of the accompanying drawings, in which like reference numerals indicate corresponding, analogous or similar elements, and in which:
**Figure 1** is a block diagram of a system for combined Positron-Emission Tomography (PET) and Magnetic Resonance Imaging (MRI), according to some embodiments of the invention;
**Figure 2A** is a schematic illustration of a system for combined Positron-Emission Tomography (PET) and Magnetic Resonance Imaging (MRI), according to some embodiments of the invention;
**Figure 2B** shows schematic illustrations of a RF coil structure of a system for combined Positron-Emission Tomography (PET) and Magnetic Resonance Imaging (MRI), according to some embodiments of the invention;
**Figure 2C** shows schematic illustrations of a PET structure of a system for combined Positron-Emission Tomography (PET) and Magnetic Resonance Imaging (MRI), according to some embodiments of the invention;
**Figure 2D** shows schematic illustrations of a radiofrequency (RF) shield of a system for combined Positron-Emission Tomography (PET) and Magnetic Resonance Imaging (MRI) including two RF shield parts, according to some embodiments of the invention;
**Figure 2E** is a schematic illustrations of a radiofrequency (RF) sleeve of a system for combined Positron-Emission Tomography (PET) and Magnetic Resonance Imaging (MRI), according to some embodiments of the invention;
**Figure 2F** and **Figure 2G** are schematic illustrations of a RF shield, RF coil structure and PET structure in a disassembled state external to a housing and in an assembled state within the housing of a system for combined Positron-Emission Tomography (PET) and Magnetic Resonance Imaging (MRI), respectively, according to some embodiments of the invention;
**Figure 3** is an example of a simultaneously acquired MRI image and PET image, and a combined PET-MRI image of a subject generated by a system for combined Positron-Emission Tomography (PET) and Magnetic Resonance Imaging (MRI), according to some embodiments of the invention; and
**Figure 4** is a flowchart of a method of generating at least one combined magnetic resonance imaging (MRI) image and Positron-Emission Tomography (PET) image, according to some embodiments of the invention.

### DETAILED DESCRIPTION OF THE INVENTION

In the following description, various aspects of the present invention are described. For purposes of explanation, specific configurations and details are set forth in order to provide a thorough understanding of the present invention. However, it will also be apparent to one skilled in the art that the present invention can be practiced without the specific details presented herein. Furthermore, well known features can have been omitted or simplified in order not to obscure the present invention. With specific reference to the drawings, it is stressed that the particulars shown are by way of example and for purposes of illustrative discussion of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention can be embodied in practice.

Before at least one embodiment of the invention is explained in detail, it is to be understood that the invention is not limited in its application to the details of construction and the arrangement of the components set forth in the following description or illustrated in the drawings. The invention is applicable to other embodiments that can be practiced or carried out in various ways as well as to combinations of the disclosed embodiments. Also, it is to be understood that the phraseology and terminology employed herein is for the purpose of description and should not be regarded as limiting.

Systems and methods for simultaneous acquisition of MRI data and PET data of a subject are disclosed. The system can include a RF coil surrounding the subject and a PET detectors array surrounding the RF coil to generate the MRI data and the PET data of the subject, respectively. The system can include a RF shield positioned between the RF coil and the PET detectors array to prevent (or substantially prevent) an interference between the RF coil and the PET detectors array to thereby allow simultaneous acquisition of the MRI data and the PET data without any one of the RF coil, the PET detectors array or the subject being moved during the imaging. The system can include an analysis unit to generate, based on the simultaneously acquired MRI data and PET data, combined PET-MRI images of the subject that include a spatially and temporally registered structural, functional and/or molecular data acquired under identical physiological conditions.

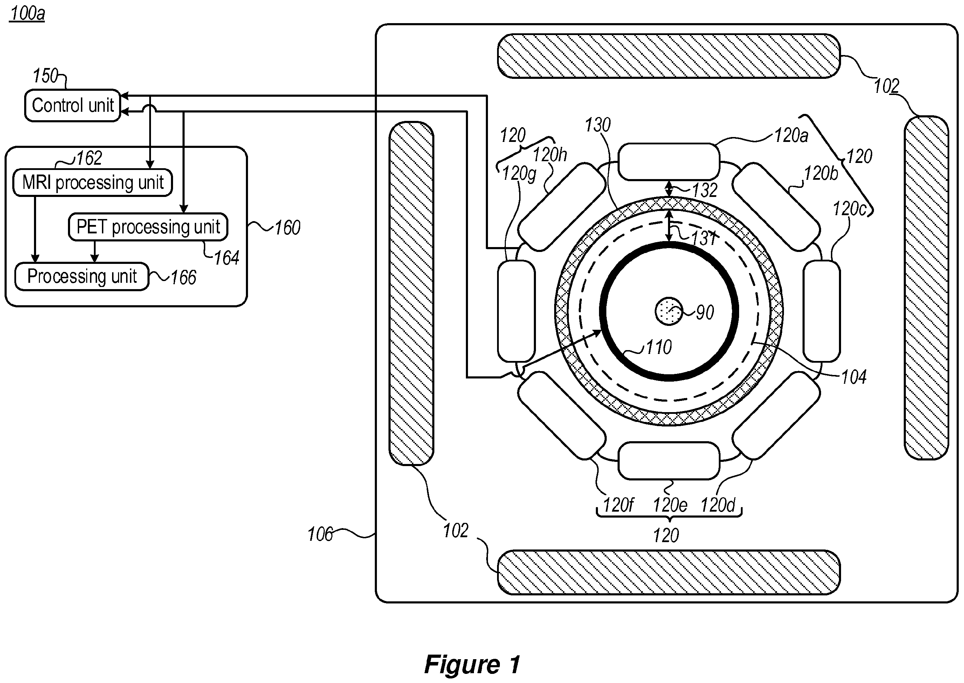

Reference is now made to **Figure 1****,** which is a block diagram of a system **100** for combined Positron-Emission Tomography (PET) and Magnetic Resonance Imaging (MRI), according to some embodiments of the invention.

System **100** can include at least one magnet **102** to generate a magnetic field within a predetermined measurement volume **104**. In various embodiments, magnets **102** include at least one of permanent magnets, superconductive magnets or a combination thereof.

System **100** can include a housing **106** to at least partly surround magnets **102**. Housing **106** can, for example, substantially eliminate a magnetic fringe field generated by magnets **102** outside housing **106**.

System **100** can include a radio frequency (RF) coil **110** (e.g., solenoid) having a RF coil bore **111**, a RF coil diameter and a RF coil length. The RF coil diameter and/or the RF coil length can have values that allow RF coil **110** to accommodate a subject **90** (e.g., a mouse), or at least a portion of subject **90**, within RF coil bore **111**. RF coil bore **111** can at least partly overlap with predetermined measurement volume **104**. In various embodiments, the RF coil diameter ranges between, for example 30-40 mm and/or the RF coil length ranges between, for example 40-55 mm.

RF coil **110** can generate an electromagnetic field within RF coil bore **111** to excite a magnetic moment of nuclei within subject **90** (or within a predetermined portion of subject **90**). RF coil **110** can be arranged to receive signals generated due to relaxation of the excited nuclei thereof. In some embodiments, system **100** includes two (or more) RF coils, for example a first RF coil and a second RF coil (not shown), wherein first RF coil generates the electromagnetic field to excite nuclei within subject **90** and second RF coil receives the signals generated due to relaxation of the nuclei thereof.

System **100** can include a Positron-Emission Tomography (PET) detectors array **120** having a PET detectors array diameter and a PET detectors array length. PET detectors array **120** can surround RF coil **110**. PET detectors array **120** can include a plurality of detectors (e.g., detectors **120a**, **120b**, **120c**, **120d**, **120e**, **120f**, **120g**, **120h**, as shown in **Figure 1**) to detect gamma rays emitted by positron-emitting radionuclides within subject **90** (or within a predetermined portion of subject **90**). For example, as is known in the PET art, positron-emitting radionuclides are injected into a subject (or the subject can swallow or inhale the radionuclides thereof) prior to taking PET images.

In various embodiments, RF coil **110** and PET detectors array **120** have a substantially annular shape and/or can be positioned concentrically (e.g., as shown in **Figure 1**). In some embodiments, RF coil **110** and PET detectors array **120** are positioned at a predetermined radial distance with respect to each other (not shown). The position of RF coil **110** and the position of PET detectors array **120** within system **100** can be determined based on, for example, a desired application and/or desired dimensions of system **100**.

System **100** can include a RF shield **130** having a RF shield diameter and a RF shield length. RF shield **130** can surround RF coil **110**. RF shield **130** can be positioned between RF coil **110** and PET detectors array **120** (e.g., as shown in **Figure 1**). In various embodiments, RF coil **110,** PET detectors array **120** and RF shield **130** are positioned to provide a first space **131** between RF coil **110** and RF shield **130** and/or to provide a second space **132** between PET **120** and RF shield **130** (e.g., as shown in **Figure 1**).

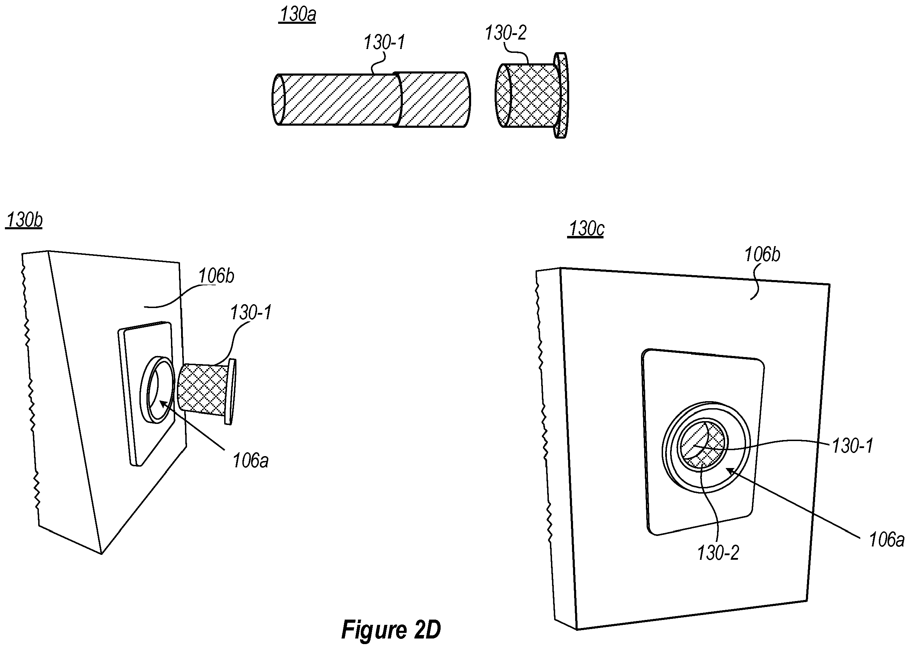

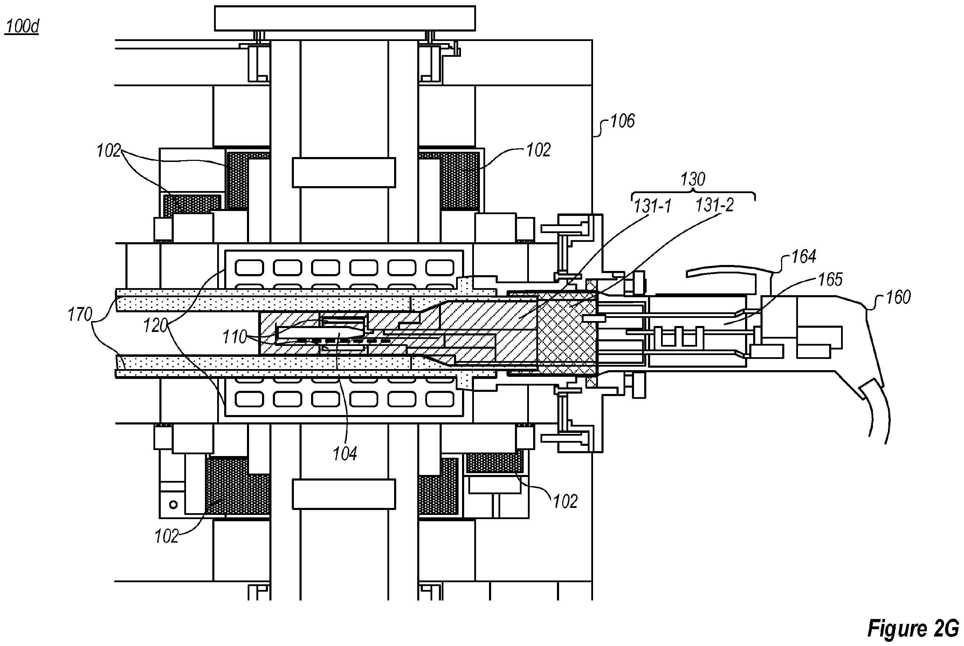

RF shield **130** can include a conductive material (e.g., copper, aluminum, and/or any other material capable of providing shielding). RF shield **130** is arranged to form a Faraday cage to provide a RF shielding (and/or electromagnetic shielding) of system **100** (e.g., as discussed below with respect to **Figure 2D** and **Figure 2G**). In some embodiments, the RF shielding includes, for example, preventing (or substantially preventing) a RF radiation generated by RF coil **110** from exiting RF coil bore **111** and preventing a RF radiation generated by PET detectors array **120** from entering RF coil bore **111**, to thereby prevent interference between RF coil **110** and PET detectors array **120.** In some embodiments, the RF shielding includes preventing (or substantially preventing) RF radiation (e.g., generated by RF coil **110)** from exiting predetermined measurement volume **104** and/or preventing external RF radiation from entering predetermined measurement volume **104**.

In various embodiments, first space **131** between RF coil **110** and RF shield **130** and/or second space **132** between PET detectors array **120** and RF shield **130** are determined to, for example, eliminate (or substantially eliminate) the RF shieling effect on the electromagnetic field generated by RF coil **110** within RF coil bore **111**, while providing the RF shielding of PET detectors array **120** and/or of system **100** (e.g., as described above). For example, first space **131** can be larger as compared to second space 132.

System **100** can include a control unit **150** in communication with RF coil **110** and with PET detectors array **120**. Control unit **150** can operate RF coil **110** and PET detectors array **120** according to a predetermined operation pattern (e.g., direct the RF coil and/or PET detector array to generate electromagnetic, magnetic fields or any combination thereof having specific intensities, directions, in accordance with operations parameters of MRI and PET as is known in the art).

In some embodiments, the predetermined operation pattern includes directing simultaneous operation of RF coil unit **110**, and thus MRI imaging, and of PET detectors array **120**, and thus PET imaging. For example, RF coil **110** can generate electromagnetic field within RF coil bore **111** and, at the same time, PET detectors array **120** can detect gamma rays emitted from subject **90**.

As is apparent to one of ordinary skill in the art, although the system can perform simultaneous PET and MRI imaging, in some embodiments, it may be desirable to operate the system in an exclusively PET or MRI mode. In these embodiments, the predetermined operation pattern can include directions for separate operation of MRI device **80** and of PET detectors array **120**. For example, RF coil **110** can generate electromagnetic field within RF coil bore **111** and, after a predetermined time delay, PET detectors array **120** can detect gamma rays emitted from subject **90,** or vice versa.

In some embodiments, PET detectors array **120** is positioned such that the electromagnetic radiation produced by RF coil **110** absent RF shield **130** impinges upon PET detectors of PET detectors array **120**. If the PET detectors of PET detectors array **120** are subject to the electromagnetic radiation from RF coil **110**, the measurements of the PET detectors of PET detectors array **120** can be erroneous and/or completely unreadable. As described above, RF shield **130** positioned between RF coil **110** and PET detectors array **120** can prevent the electromagnetic radiation from coil **110** from ruining PET detectors array **120** measurements because, for example, PET detectors array **120** is shielded. In this manner, simultaneous PET imaging and MRI imaging can occur, without, for example, any one of RF coil **110**, PET detectors array **120** and/or subject **90** being moved during an imaging procedure.

System **100** can include an analysis unit **160**. Analysis unit **160** can include a MRI processing unit **162**. MRI processing unit **162** can receive MRI data (e.g., signals received by RF coil **110**) and generate, based on the MRI data, one or more MRI images of subject **90** (or at least a portion of subject **90**). In various embodiments, the MRI images include at least one of a set of temporary sequential MRI images of subject **90**, a set of spatial MRI images of subject **90** or any combination thereof.

Analysis unit **160** can include a PET processing unit **164**. PET processing unit **164** can receive PET data (e.g., received by PET detectors array **120**) and generate, based on the PET data, one or more PET images of subject **90** (or at least a portion of subject **90**). In various embodiments, the PET images include at least one of a set of temporary sequential PET images of subject **90**, a set of spatial PET images of subject **90** or any combination thereof.

Analysis unit **160** can include a processing unit **166**. Processing unit **166** can receive the MRI images (e.g., from MRI processing unit **162**) and the PET images (e.g., from PET processing unit **164**). In various embodiments, the MRI images and/or the PET images are generated external to analysis unit **160**, while analysis unit **160** can be adapted to receive the externally generated MRI images and PET images. Processing unit **166** can combine at least one of the MRI images with at least one of the PET images to thereby generate at least one combined PET-MRI image.

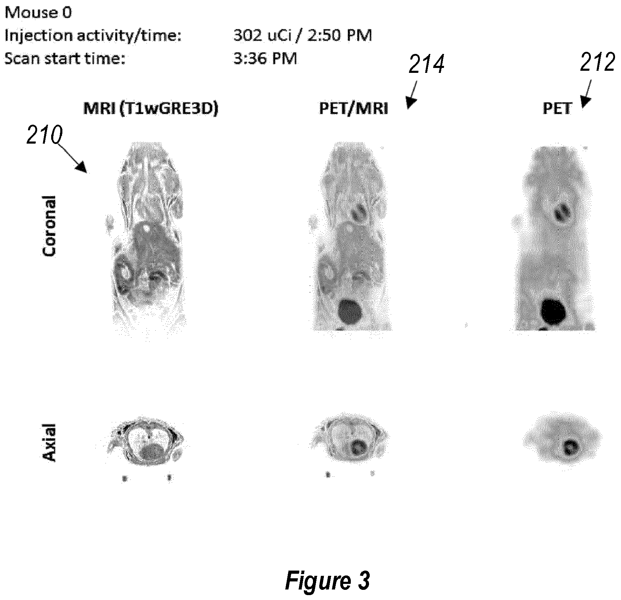

In various embodiments, the combining includes any one of registering, superimposing and/or fusing the MRI images and the PET images. In various embodiments, the combined images include spatially registered and/or temporally registered MRI images and PET images of subject **90** (or at least a portion of subject 90) acquired under identical physiological conditions. An example of combined PET-MRI image (e.g., generated by system **100)** is described below with respect to **Figure 3**.

Alternatively or complementarily, system **100** includes other detectors (not shown) that are not influenced by RF shielding generated by RF shield **130** (e.g., as described above). For example, system **100** can include optical detectors positioned external to RF coil bore **111** (not shown). In this case, RF shield **130** can include openings at predetermined locations along RF shield **130**, to, for example, enable detection of photons emitted from subject **90** by the optical detectors thereof. Analysis unit **160** can be further arranged to generate at least one optical image (e.g., based on optical data received from the optical detectors) and further to combine the at least one optical image with the at least one MRI image and/or with the at least one PET image (e.g., as described above).

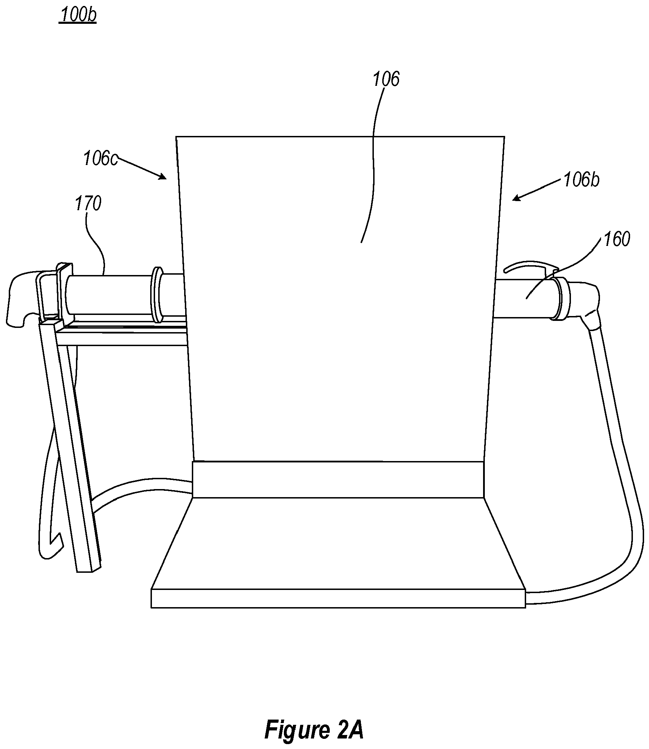

Reference is now made to **Figure 2A**, which is a schematic illustration of a system for combined Positron-Emission Tomography (PET) and Magnetic Resonance Imaging (MRI), such as system **100,** according to some embodiments of the invention.

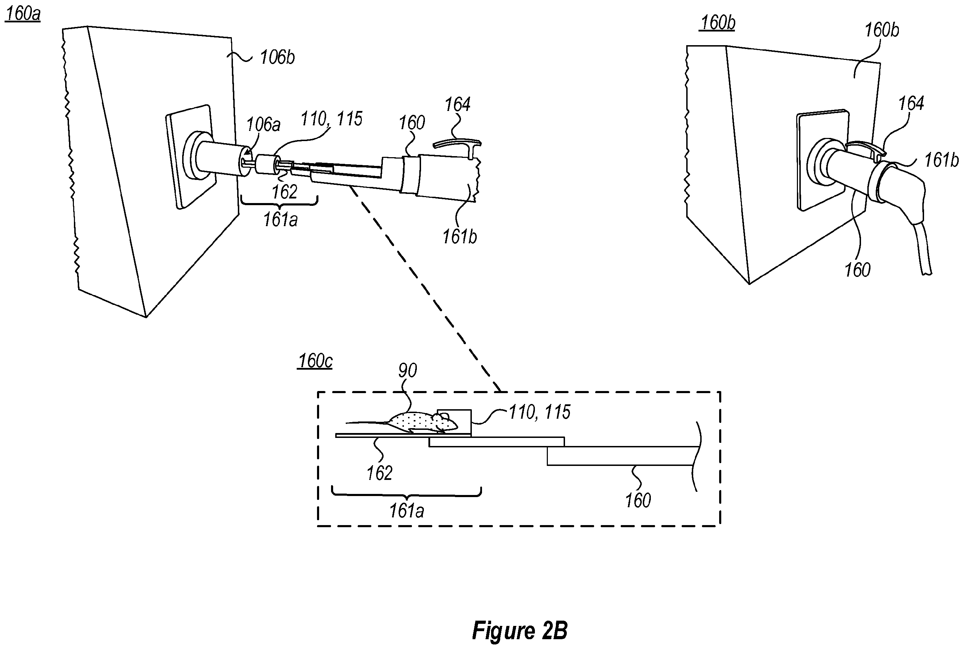

System **100** can include a RF coil structure **160** and/or PET structure **170** (e.g., as shown in **Figure 2A**). RF coil structure **160** and/or PET structure **170** can be used to, for example, insert RF coil **110** accommodating subject **90** and PET detectors array **120** into measurement volume **104** (e.g., within housing **106**) of system **100** (e.g., as described below with respect to **Figures 2B-2C**).

Reference is now made to **Figure 2B**, which shows schematic illustrations of a RF coil structure, such as RF coil structure **160**, of a system for combined Positron-Emission Tomography (PET) and Magnetic Resonance Imaging (MRI), such as system **100**, according to some embodiments of the invention.

Illustrations **160a** and **160b** in **Figure 2B** show a RF coil structure **160** positioned external to housing **160** (e.g., in a non-operating position) and inside housing **106** (e.g., in an operating position), respectively. Illustration **160c** in **Figure 2B** shows a cradle **162** of RF coil structure **160** accommodating subject **90**.

RF coil structure **160** can include, at RF coil structure's distal end **161a**, a cradle **162**. Cradle **162** can receive and accommodate a subject **90**, for example a mouse.

In various embodiments, RF coil **110** is mounted on, or embedded within, a RF coil support **115** (e.g., as shown in **Figure 2B**). RF coil support **115** can be mounted on cradle **162** such that RF coil **110** surrounds cradle **162**. For example, RF coil support **110** can be thread onto cradle **162** such that RF coil bore **111** accommodates both subject **90** and cradle **162**.

RF coil structure **160** can be inserted into housing **106** via, for example, an opening **106a** on a front face **106b** of housing **106**. A RF coil structure's **160** length and/or cradle's **162** length can be determined to position cradle **162**, RF coil **110** and subject **90** within predetermined measurement volume **104** within housing **106**.

RF coil structure **160** can include a handle **164** affixed to a RF coil structure's proximal end **161b** to, for example, insert and/or remove RF coil structure **160** into/from housing **106**. RF coil structure **160** can be arranged to route RF coil's **110** wiring and/or medical tubing connected to subject **90** external to housing **106**, e.g., through an interior **165** of RF coil structure **160** (e.g., as shown in **Figure 2G****),** while preventing (or substantially preventing) an external RF radiation from entering predetermined measurement volume **104** and/or preventing (or substantially preventing) RF radiation from exiting predetermined measurement volume **104**.

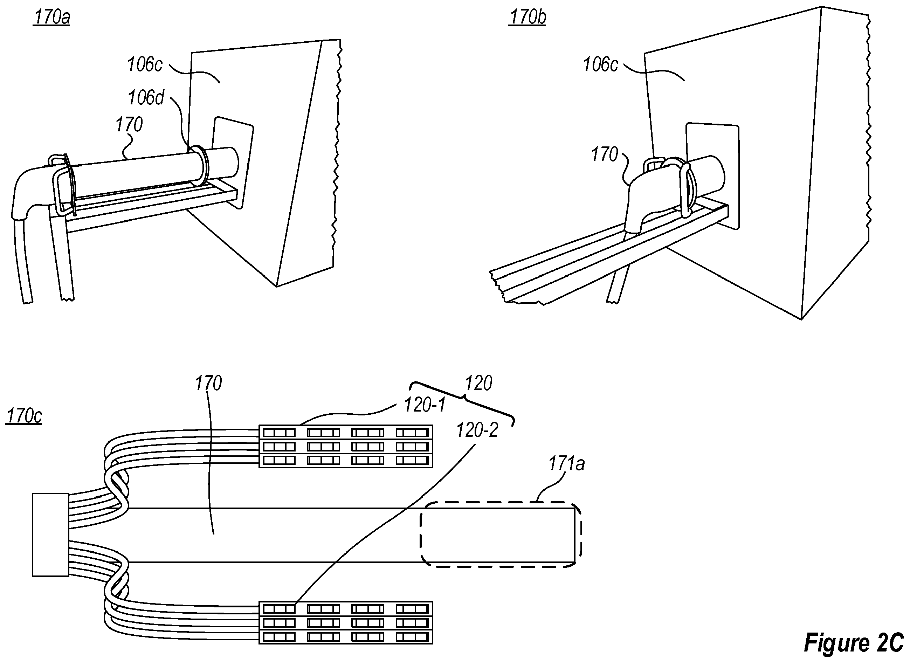

Reference is now made to **Figure 2C****,** which shows schematic illustrations of a PET structure, such as PET structure **170**, of a system for combined Positron-Emission Tomography (PET) and Magnetic Resonance Imaging (MRI), such as system **100**, according to some embodiments of the invention.

Illustrations **170a** and **170b** in **Figure 2C** show a PET structure **170** positioned partly external to housing **106** (e.g., in a non-operating position) and inside housing **106** (e.g., in an operating position), respectively. Illustration **170c** in **Figure 2C** shows PET detectors array **120** and PET structure **170** external to housing **106**.

PET detectors array **120** can include one or more PET detectors array parts that can be mounted on a distal portion **171a** of PET structure **170**. For example, PET detectors array **120** can include a first PET detectors array part **120-1** and a second PET detectors array part **120-2** (e.g., as shown in **Figure 2C**).

PET structure **170** can be inserted into housing **106** via, for example, an opening **106d** on a back face **106c** of housing **106.** A PET structure's **170** length can be determined to position PET detectors array **120** mounted on PET structure's distal portion **171a** within predetermined measurement volume **104** to thereby surround RF coil **110** and RF shield **130** (e.g., as described above with respect to **Figure 1**).

PET structure **170** can be arranged to route PET detectors array's **120** wiring external to housing **106** (e.g., through an interior of PET structure **170**) while preventing (or substantially preventing) an external RF radiation from entering predetermined measurement volume **104** and/or preventing (or substantially preventing) RF radiation from exiting predetermined measurement volume **104.**

Reference is now made to **Figure 2D**, which shows schematic illustrations of a radiofrequency (RF) shield, such as RF shield **130,** of a system for combined Positron-Emission Tomography (PET) and Magnetic Resonance Imaging (MRI), such as system **100,** including two RF shield parts **130-1**, **130-2**, according to some embodiments of the invention.

Illustration **130a** in **Figure 2D** shows a first RF shield part **130-1** and a second RF shield part **130-2**. Illustration **130b** in **Figure 2D** shows a second RF shield part **130-2** external to housing **106.** Illustration **130c** in **Figure 2D** shows first RF shield part **130-1** and second RF shield part **130-2** within housing **106.**

In some embodiments, RF shield **130** includes two RF shield parts, for example, a first RF shield part **130-1** and a second RF shield part **130-2** (e.g., as shown in **Figure 2D**). Each of first RF shield part **130-1** and second RF shield part **130-1** can include conductive material (e.g., copper). For example, each of first RF shield part **130-1** and/or second RF shield part **130-2** can be a copper cylinder and/or can be a substantially annular sleeve that can include a copper mesh.

In some embodiments, RF structure **160** is arranged to electrically connect, upon insertion into housing **106,** to first RF shield part **130-1** and to second RF shield part **130-2** (e.g., as shown below in **Figure 2G****)** to form a Faraday cage to thereby provide RF shielding of system **100.** The RF shielding can include, for example, preventing (or substantially preventing) RF radiation (e.g., generated by RF coil **110**) from exiting predetermined measurement volume **104** and preventing (or substantially preventing) external RF radiation from entering predetermined measurement volume **104** and/or preventing (or substantially preventing) interference between RF coil **110** and PET detectors **120** (e.g., as described above with respect to **Figure 1**).

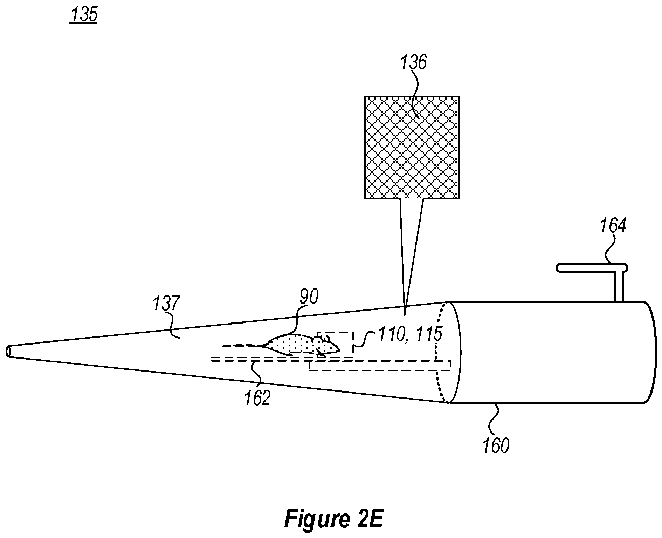

Reference is now made to **Figure 2E**, which is a schematic illustrations of a radiofrequency (RF) sleeve **135** of a system for combined Positron-Emission Tomography (PET) and Magnetic Resonance Imaging (MRI), such as system **100**, according to some embodiments of the invention.

System **100** can include a RF sleeve **135**. RF sleeve **135** can include a metal mesh **136** (e.g., made of conductive material, such as copper). Metal mesh **136** can be embedded within, for example, fabric material **137**. In some embodiments, RF sleeve **135** envelopes cradle **162** of RF structure **160** accommodating RF coil **110** and subject **90**. RF sleeve **135** can be electrically connected to RF structure **160** to form a Faraday cage to thereby provide additional RF shielding (e.g., additional to RF shielding provided by RF shield **130)** of RF coil **110** and PET detectors **120** (e.g., as described above with respect to **Figure 1****)**.

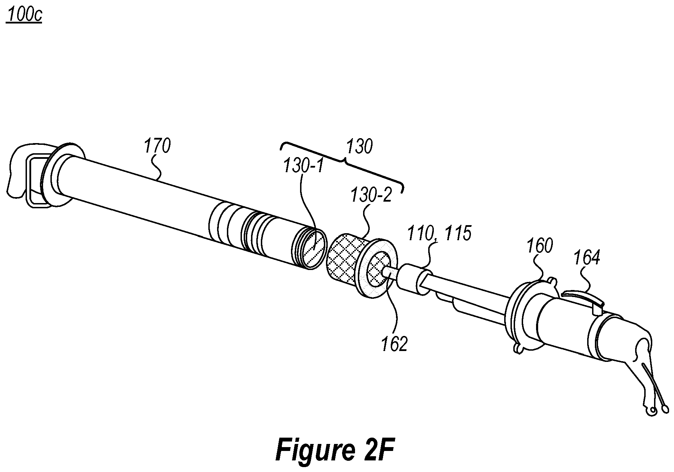

Reference is now made to **Figure 2F** and **Figure 2G**, which are schematic illustrations of a RF shield **130**, RF coil structure **160** and PET structure **170** in a disassembled state external to housing **106** and in an assembled state within housing **106** of a system for combined Positron-Emission Tomography (PET) and Magnetic Resonance Imaging (MRI), such as system **100**, respectively, according to some embodiments of the invention. Illustration **100c** in **Figure 2F** shows a perspective view and illustration **100d** in **Figure 2G** shows a cross-sectional view.

Positioning ofRF coil structure **160** into operating position (e.g., as described above with respect to **Figure 2B****)** can locate cradle **162** and RF coil **110** (e.g., surrounding subject **90**) within predetermined measurement volume **104**. Positioning of PET structure **170** into operating position (e.g., as described above with respect to **Figure 2C**) can locate PET detectors **120** to surround RF coil **110** and RF shield **130** (e.g., first RF shield part **130-1**, as shown in **Figure 2G**) along predetermined measurement volume **104.**

Upon positioning of RF coil structure **160** and/or of PET structure **170** into the operative positions thereof, RF coil structure **160**, PET structure **170**, first RF shield part **130-1**and/or second RF shield part **130-2** can be electrically connected (e.g., as shown in **Figure 2G**) to provide the Faraday cage (e.g., as described above with respect to **Figure 2D**). The Faraday cage can provide RF shielding of system **100** to thereby prevent (or substantially prevent) RF radiation (e.g., generated by RF coil **110**) from exiting predetermined measurement volume **104** and prevent external RF radiation from entering predetermined measurement volume **104** and/or prevent (or substantially prevent) interference between RF coil **110** and PET detectors **120** (e.g., as described above with respect to **Figure 1** and **Figure 2D**).

In some embodiments, elements of system **100** (e.g., as described above with respect to **Figure 1** and **Figures 2A****-2G**) are scaled in size for accommodation of an adult human, a portion of the adult human (e.g., a head), a human infant, a portion of the human infant (e.g., a head) and/or laboratory animals (e.g., rats, pigs, etc.).

Reference is now made to **Figure 3**, which is an example of a simultaneously acquired MRI image **210** and PET image **212**, and a combined PET-MRI image **214** of a subject **90**, such as mouse, generated by a system for combined Positron-Emission Tomography (PET) and Magnetic Resonance Imaging (MRI), such as system **100,** according to some embodiments of the invention.

Combining of simultaneously acquired MRI images **210** and PET images **212** into combined PET-MRI image **214** (e.g., as described above with respect to **Figure 1**) allows to determine structural data (e.g., from MRI images) and functional (and/or molecular) data (e.g., from PET images **214**) relating to subject **90**, wherein the structural and the functional data is spatially and temporally registered and acquired under identical physiological conditions.

Some embodiments of the present invention can include a device for combined PET-MRI, which can be operative in a MRI device. For example, the MRI device (e.g., that can utilize superconductive magnets, permanent magnets and/or a combination thereof to generate a magnetic field) can be retrofitted to receive the device and to operate in combination with the device.

The device can include a RF coil having a RF coil bore that at least partly overlaps with a predetermined measurement volume of the MRI device and having a diameter to accommodate at least a portion of a subject (e.g., RF coil **110**, as described above with respect to **Figure 1** and **Figure 2B**). The RF coil can generate an electromagnetic field within the RF coil bore to excite nuclei within the at least portion of the subject and to receive signals emitted from the at least portion of the subject when the electromagnetic field is removed and the excited nuclei relax.

The device can include a PET detectors array surrounding the RF coil and to detect gamma rays emitted by positron-emitting (PE) radionuclides within the subject (e.g., PET detectors array **120,** as described above with respect to **Figure 1** and **Figure 2C**).

The device can include a RF shield surrounding the RF coil and positioned between the RF coil and the PET detectors array (e.g., RF shield **130**, as described above with respect to **Figure 1**, **Figures 2D-2E** and **Figure 2G**).

In various embodiments, the device includes features from different embodiments of system **100** described above with respect to **Figure 1** and **Figures 2A****-2G**. For example, the device can include a RF coil structure (e.g., RF coil structure **160**, as described above with respect to **Figure 2B**) and a PET structure (e.g., PET structure **170**, as described above with respect to **Figure 2C**) to thereby enable insertion of the device within the predetermined measurement volume of the MRI device; and/or the device can include an analysis unit (e.g., analysis unit **160**, as described above with respect to **Figure 1**), to generate combined PET MRI images (e.g., as described above with respect to **Figure 1** and **Figure 3**).

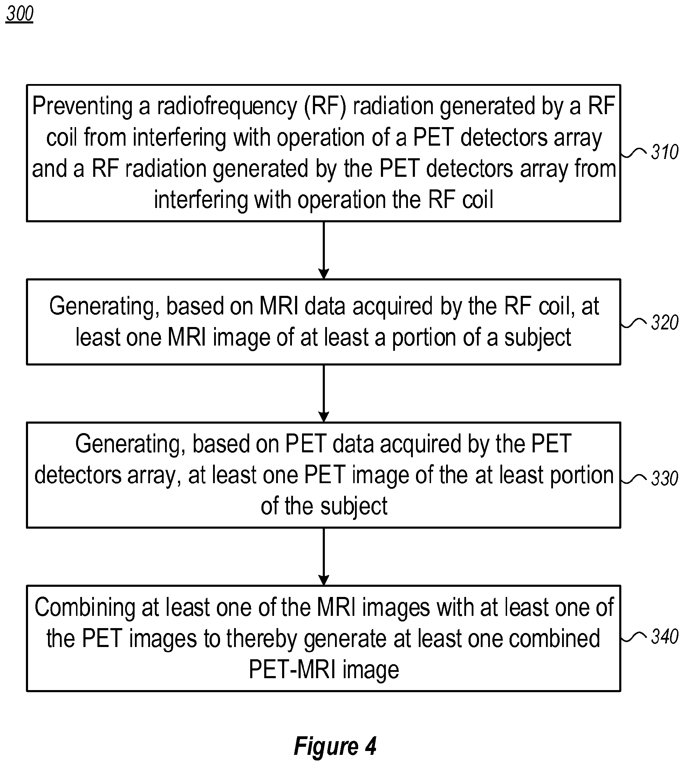

Reference is now made to **Figure 4**, which is a flowchart of a method **300** of generating at least one combined magnetic resonance imaging (MRI) image and Positron-Emission Tomography (PET) image, according to some embodiments of the invention. Method **300** can be implemented by system **100**, which may be configured to implement method **300**.

Method **300** can include preventing **310** a radio frequency (RF) radiation generated by a RF coil from interfering with operation of a PET detectors array and a RF radiation generated by the PET detectors array from interfering with operation the RF coil. For example, an RF shield can be positioned between the RF coil and the PET detectors array (e.g., as described above with respect to **Figure 1**, **Figures 2D-2E** and **Figure 2G**).

Method **300** can include generating **320** (e.g., by analysis unit **160,** as described above with respect to **Figure 1**), based on MRI data acquired by the RF coil, at least one MRI image (e.g., MRI image **210**, as described above with respect to **Figure 3**) of at least portion of a subject.

Method **300** can include generating **330** (e.g., by analysis unit **160**, as described above with respect to **Figure** 1), based on PET data acquired by the PET detectors array, at least one PET image (e.g., PET image **212,** as described above with respect to **Figure 3****)** of the at least portion of the subject.

Method **300** can include combining **340** (e.g., by analysis unit **160**, as described above with respect to **Figure 1**) at least one of the MRI images with at least one of the PET images to thereby generate at least one combined PET-MRI image (e.g., combined PET-MRI image **214**, as described above with respect to **Figure 3**).

In various embodiments, method **300** includes operating the RF coil and the PET detectors array simultaneously (e.g., at the same time) or separately (e.g., at a predetermined time-delay with respect to each other) without any one of the RF coil, the PET detectors array or the subject being moved (e.g., as described above with respect to **Figure 1**).

In various embodiments, the combining includes at least one of registering, superimposing, fusing or any combination thereof of at least one of the MRI images and at least one of the PET images. In various embodiments, the combined PET-MRI images include spatially registered and/or temporally registered MRI images and PET images of the subject (or at least a portion of the subject) acquired under identical physiological conditions (e.g., as described above with respect to **Figure 1**).

One advantage of some embodiments of the present invention is that they allow simultaneous acquisition of MRI data of a subject, e.g., by a RF coil surrounding the subject, and PET data of the subject, e.g., by a PET detectors array surrounding the RF coil, without moving any one of the RF coils, the PET detectors array or the subject during an imaging procedure. Acquiring the MRI data and the PET data simultaneously (e.g., at the same time) can be achieved due to a RF shield surrounding the RF coil and positioned between the RF coil and the PET detectors array, which prevents an interference between the RF coil and the PET detectors array thereof. The simultaneous acquisition of the MRI data and the PET data allows determining a spatially and temporally registered structural, functional and/or molecular data acquired under identical physiological conditions.

In the above description, an embodiment is an example or implementation of the invention. The various appearances of "one embodiment", "an embodiment", "certain embodiments" or "some embodiments" do not necessarily all refer to the same embodiments. Although various features of the invention can be described in the context of a single embodiment, the features can also be provided separately or in any suitable combination. Conversely, although the invention can be described herein in the context of separate embodiments for clarity, the invention can also be implemented in a single embodiment. Certain embodiments of the invention can include features from different embodiments disclosed above, and certain embodiments can incorporate elements from other embodiments disclosed above. The disclosure of elements of the invention in the context of a specific embodiment is not to be taken as limiting their use in the specific embodiment alone. Furthermore, it is to be understood that the invention can be carried out or practiced in various ways and that the invention can be implemented in certain embodiments other than the ones outlined in the description above.

The invention is not limited to those diagrams or to the corresponding descriptions. For example, flow need not move through each illustrated box or state, or in exactly the same order as illustrated and described. Meanings of technical and scientific terms used herein are to be commonly understood as by one of ordinary skill in the art to which the invention belongs, unless otherwise defined. While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention should not be limited by what has thus far been described, but by the appended claims and their legal equivalents.

## Claims

1. A device for use in a magnetic resonance imaging (MRI) device for combined MRI and Positron-Emission Tomography (PET), the device comprising:
a radio frequency (RF) coil having a RF coil bore to at least partly overlap a predetermined measurement volume of the MRI device in use, and having a diameter to accommodate at least a portion of a subject, the RF coil to generate an electromagnetic field within the RF coil bore to excite nuclei within the at least portion of the subject and to receive signals emitted from the at least portion of the subject when the electromagnetic field is removed and the excited nuclei relax;
a PET detectors array surrounding the RF coil and to detect gamma rays emitted by positron-emitting (PE) radionuclides within the subject; and
a RF shield surrounding the RF coil and positioned between the RF coil and the PET detectors array.

2. The device of claim 1, further comprising an analysis unit in communication with at least one of the RF coil and the PET detectors array, the analysis unit to:
generate, based on MRI data acquired by the RF coil, at least one MRI image of the at least portion of the subject;
generate, based on PET data acquired by the PET detectors array, at least one PET image of the at least portion of the subject; and
combine at least one of the MRI images with at least one of the PET images to thereby generate at least one combined PET-MRI image.

3. The device of claim 2, wherein each of the at least one MRI image and the at least one PET image comprises at least one of a set of temporary sequential images, a set of spatial images or any combination thereof.

4. The device of claim 2 or claim 3, configured to perform the combining by at least one of registering, superimposing, fusing or any combination thereof of at least one of the MRI images and at least one of the PET images.

5. The device of any preceding claim, wherein the RF coil and the detector array are positioned such that the subject, RF coil and the detector array are stationary during operation.

6. The device of any preceding claim, wherein the RF shield comprises at least one of a cylinder or a mesh made from a conductive material.

7. The device of any preceding claim, wherein the RF shield to form a Faraday cage to thereby provide RF shielding of the system.

8. The device of any preceding claim, wherein the RF coil and the PET detectors array are positioned concentrically or at a predetermined radial distance with respect to each other.

9. A system for combined magnetic resonance imaging (MRI) and Positron-Emission Tomography (PET), the system comprising:
at least one magnet to generate a magnetic field within a predetermined measurement volume; and
a device according to any preceding claim.

10. The system of claim 9, wherein the MRI device comprises at least one of permanent magnets, superconductive magnets or a combination thereof to generate a magnetic field within the predetermined measurement volume.

11. A method of generating at least one combined magnetic resonance imaging (MRI) image and Positron-Emission Tomography (PET) image, the method comprising:
preventing a radiofrequency (RF) radiation generated by a RF coil from interfering with operation of a PET detectors array and a RF radiation generated by the PET detectors array from interfering with operation the RF coil;
generating, based on MRI data acquired by the RF coil, at least one MRI image of at least a portion of a subject;
generating, based on PET data acquired by the PET detectors array, at least one PET image of the at least portion of the subject; and
combining at least one of the MRI images with at least one of the PET images to thereby generate at least one combined PET-MRI image.

12. The method of claim 11, further comprising operating the RF coil and the detector array simultaneously or separately without any one of the RF coil, the PET detectors array or the subject being moved during an imaging procedure.

13. The method of claim 11 or claim 12, wherein the combining comprises at least one of registering, superimposing, fusing or any combination thereof of at least one of the MRI images and at least one of the PET images.
